# EUROPEAN PATENT SPECIFICATION

(11) **EP 0 574 414 B1**
(45) Date of publication and mention of the grant of the patent: **14.07.1999**
(21) Application number: 92903981.6
(22) Date of filing: 13.01.1992
(51) Int. Cl.: C07K 14/475, C07K 14/705, C12N 15/12, C12N 5/10, C12N 1/21, C12N 1/38, A61K 38/17, A61K 39/395

(54) **LIGAND GROWTH FACTORS THAT BIND TO THE erbB-2 RECEPTOR PROTEIN AND INDUCE CELLULAR RESPONSES**
WACHSTUMSFAKTOR-LIGAND, DER AN DEN ERBB-2-REZEPTOR BINDET UND DIE ZELLULÄREN REAKTIONEN INDUZIERT
FACTEURS DE CROISSANCE LIGANDS QUI SE LIENT AU RECEPTEUR PROTEIQUE erbB-2 ET INDUISENT DES REPONSES CELLULAIRES

(30) Priority: 14.01.1991 US 640497
(43) Date of publication of application: 22.12.1993
(73) Proprietor: GEORGETOWN UNIVERSITY, Washington, D.C. 20057 (US)
(72) Inventor: LIPPMAN, Marc, E., Bethesda, MD 20817 (US); LUPU, Ruth, Rockville, MD 20850 (US)
(74) Representative: Dean, John Paul
(86) International application number: US9200329
(87) International publication number: WO9212174

(56) References cited:
- WO-A-91/15230
- US-A- 5 030 565
- Proc. Natl. Acad. Sci. USA, Volume 86, issued May 1989, Y. YARDEN et al., "Experimental approaches to hypothetical hormones: Detection of a candidate ligang of the neu protooncogene", pages 3179-3183, see pages 3179, 3181, 3182.
- Science, Volume 249, issued 28 September 1990, R. LUPU et al., "Direct Interaction of Ligand for the erbB2 Oncogene Product with EGF Receptor and p185er bB2", pages 1552-1555, see page 1552.
- Cell Growth & Diferrentation, Volume 1, issued December 1990, O. ALPER, "The Presence of c-erbB-2 Gene Product-related Protein in Culture Medium Conditioned by Breast Cancer Cell line SK-BR-3", pages 591-599, see page 591, 596, 597.
- Proc. Natl. Acad. Sci. USA, Volume 88, issued March 1991, N.J. MAIHLE et al., "Native avian c-erbB gene expresses a secreted protein product corresponding to the ligand-binding domain of the receptor", pages 1825-1829.
- Cancer Research, Volume 51, issued 15 May 1991, B.C. LANGTON et al., "An antigen Immunologically Related to the External Domain of gp185 is Shed from Nude Mouse Tumors Overexpressing the c-erbB-2(HER-2 /neu) Oncogene", pages 2593-2598.
- Virology, Volume 152, issued 1986, L.E. GENTRY et al., "Characterization of Site-specific Antibodies to the erbB Gene Product and EGF Receptor: Inhibition of Tyrosine Kinase Activity", pages 421-431, see pages 421, 424, 426.
- Virology, Volume 111, issued 1981, H. BEUG et al., "Production and Characterization of Antisera Specific for the erb-Portion of p75, the Presumptive Transforming Protein of Avian Erythroblastosis Virus", pages 201-210.

## Description

### Field of the Invention

The invention relates to the field of ligand-growth factor receptor interactions and the cellular responses induced by such interactions. Specifically, a ligand is described which is capable of binding exclusively to the erbB-2 transmembrane protein. The present invention additionally relates to anti-ligand molecules capable of recognising and binding to the erbB-2 ligand molecule and to screening assays for such ligands. The present invention further relates to uses for the erbB-2 ligand, the anti-ligand molecules and the screening assay. Furthermore, the invention relates to a cloned gene capable of expressing the erbB-2 ligand of the present invention.

### Background of the Invention

Transforming growth factor ligands belong to a family of heat and acid-stable polypeptides which allow cells to assume a transformed morpholoy and form progressively growing colonies in anchorage-independent growth assays (DeLarco *et al., Proc. Natl. Acad. Sci. USA,* **75**:4001-4005 (1978); Moses *et al., Cancer Res.,* **41**:2842-2848 (1981); Ozanne *et al., J. Cell. Physiol.,* **105**:163-180 (1980); Roberts *et al., Proc. Natl. Acad. Sci. USA.,* **77**:3494-3498 (1980)). A proteinaceous substance bindable with p185 which affects the growth of cells expressing p185 is known from WO91/15230. The epidermal growth factor receptor (EGFR) and its physiologic ligands, epidermal growth factor (EGF) and transforming growth factor α (TGFα), play a prominent role in the growth regulation of many normal and maligant cell types (Carpenter G., *Annu. Rev., Biochem.,* **56**:881-914 (1987)).

One role the EGF receptor system may play in the oncogenic growth of cells is through autocrine-stimulated growth. If cells express the EGFR and secrete EGF and/or TGFα then such cells could stimulate their own growth. Since some human breast cancer cell lines and tumors express EGFR (Osborne et al., J. Clin. Endo. Metab., 55:86-93 (1982) ; Fitzpatrick et al., Cancer Res., 44:3442-3447 (1984); Filmus et al., Biochem. Biophvs. Res. Commun., 128:898-905 (1985); Davidson et al., Mol. Endocrinol, 1:216-223 (1987); Sainsbury et al., Lancet, i:1398-1402 (1987); Perez et al., Cancer Res. Treat., 4:189-193 (1984)) and secrete TGFα (Bates et al., Cancer Res., 46:1707-1713 (1986); Bates et al., Mol. Endocrinol, 2:543-555 (1988)) an autocrine growth stimulatory pathway has been proposed in breast cancer (Lippman et al., Breast Cancer Res. Treat., 7:59-70 (1986)).

An analogous autocrine growth stimulatory pathway proposed for epidermal growth factor receptor and its ligands may also be employed by a growing list of oncogene encoded transmembrane proteins that have structure reminiscent of growth factor receptors. This list includes the protooncogenes neu and its human equivalent erbB-2 or HER2 (Bargmann et al., Nature, 319:226-229 (1986); Coussens et al., Science, 230:1131-1139 (1985) ; Yamamoto et al., Nature, 319:230-234 (1986); c-kit (Yarden et al., EMBO, 6:341-3351 (9187); ros (Neckameyer et al., Mol. Cell. Biol. 6:1478-1486 (1986); met (Park et al., PNAS, 84:6379-6383 (1987); trk (Martin-Zanca et al., Nature, 319:743-748 (1986); and ret (Takahashi et al., Mol. Cell. Biol., 7:1378-1385 (1987)). The erbB-2 and c-kit protooncogenes encode factors that display remarkable structural homology with EGFR (Yarden et al., Annu. Rev. Biochem., 57:443-478 (1988). Although erbB-2 and its related oncogene neu are related to EGFR, these proteins are distinct. For example, known EGFR ligands such as EGF and TGFα do not bind to erbB-2 receptor. (King et al., EMBO, 7:1647 (1988); and Stern et al., EMBO, 7:995 (1988).

If, according to the autocrine growth stimulatory pathway, malignant cells are capable of secreting a potent tumor growth factor in vivo, it is plausible that the growth factor ligand might be detected in body fluids, much like human chorionic gonadotropin or α-fetoprotein, and could be used as a tumor marker and a prognostic variable. Studies suggest that TGFα activity can be detected in body fluids of cancer patients and that its presence may provide important information concerning the biology of a patient's tumor (Stromberg et al., J. Cell. Biochem., 32:247-259 (1986); Twardzick et al., J. Natl. Cancer Inst., 69:793-798 (1982) ; Sherwin et al., Cancer Res., 43:403-407 (1983)).

The erbB-2 protooncogene amplification has been found in breast, ovarian, gastric, salivary gland, and in non-small cell carcinomas of the lung (King et al., Science, 229:974 (1985); Slamon et al. , Science, 244:707 (1989); Yokota et al., Lancet, 1:765 (1986); Fukushige et al., Mol. Cell. Biol., 6:955 (1986); Semba et al., Proc. Natl. Acad. Sci. USA, 82:6497 (1985); Weiner et al., Cancer Res., 50:421 (1990)). Amplification and/or overexpression of the erbB-2 protooncogene has been found to correlate with poor prognosis in breast, ovarian and non-small cell lung carcinomas (Slamon et al., Science, 235:177 (1986); Slamon et al., Science, 244:707 (1989); Guerin et al., Oncogene Research, 3:21 (1988); Wright et al., Cancer Res., 49:2087 (1989); Kern et al., Cancer Res., 50:5184 (1990); DiFiore et al., Science, 237:178 (1987)). In addition to these clinical studies, in vitro studies strongly suggest that overexpression of the erbB-2 transmembrane receptor (p185^{erbB-2}) may have an important role in tumor progression (DiFiore et *al., Science,* **237**:178 (1987); Hudziak *et al., Proc. Natl. Sci. USA,* **84**:7159 (1987)).

Although ligands for EGFR are known, namely EGF and TFGα, few ligands for the oncogene encoding transmembrane proteins such as erbB-2, *ros, met* etc. have been characterised. Lupu *et al., Science,* **249**:1552-1555 (1990) identified a 30 kilodalton (kDa) glycoprotein (gp30) which is similar to TGFα in its ability to bind to the EGFR, phosphorylate EGFR, and induce colony formation. Direct binding of the gp30 to p185^{erB-2} was confirmed by binding competition experiments, suggesting that gp30 is a ligand for p185^{erbB-2}. Thus, Lupu *et al.* identified and characterised a 30 kDa ligand that binds both EGFR and erbB-2 receptor.

At present, no ligand is known which binds to the erbB-2 transmembrane protein (p185^{erbB-2}) but fails to react with EGFR. Such a ligand will be important for understanding the function of p185^{erB-2} and may be a potential therapeutic and diagnostic target for noeplasia.

### Summary of the Invention

The present invention relates to a substantially pure protein, wherein said protein:
reverses Mab 4D5-dependent inhibition of erbB-2 overexpressing cells;
reverses antiproliferative effect of soluble erbB-2 extracellular domain (ECD);
inhibits cell proliferation and colony formation of cells which overexpress erbB-2;
has an apparent molecular weight as measured by SDS PAGE of about 75 kDa;
is capable of inducing phosphorylation of p185^{erbB-2}; and
does not bind EGFR. Methods of obtaining the purified ligands of the present invention are also included in the present invention.

The invention additionally pertains to anti-ligand molecules such an antibodies or fragments of antibodies and blocking peptides which bind to the erbB-2 ligand of the present invention. A method to detect the presence of cells which express the erbB-2 ligand with these anti-ligand molecules is also disclosed. A further aspect of the invention involves the use of the erbB-2 ligand to detect cells expressing p185^{erbB-2} transmembrane protein.

The invention further pertains to a recombinant DNA molecule coding for a gene which is capable of expressing the erbB-2 ligand of the present invention and to host cells which contain such a recombinant DNA molecule.

The invention is also directed to a method for treating a number of cancers associated with erbB-2 transmembrane protein overexpression including breast, ovarian, gastric, lung, prostate, salivary gland and thyroid carcinomas.

### Description of the Drawings

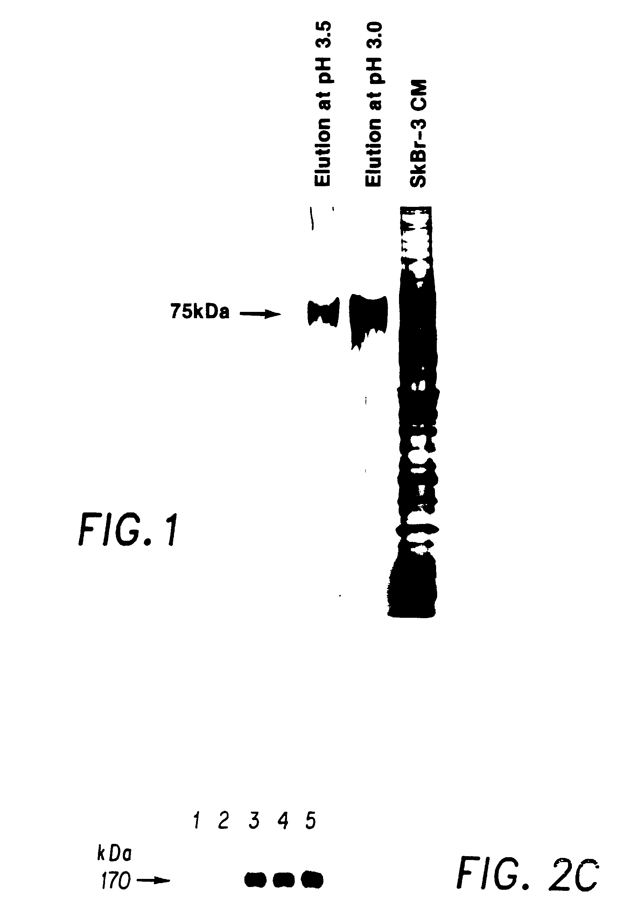

Figure 1 shows SDS-polyacrylamide gel electrophoresis of samples eluted from an affinity column coupled to p185^{erbB-2} extracellular domain. erbB-2 extracellular domain (ECD) was coupled to polyacrylamide-hydrazide sepharose beads (Sigma). After extensive washes of the beads (5 volumes) with ice-cold 1.0 M HCl, the beads were activated with 0.5 M NaNO₂ (1 volume). The temperature was maintained at 0°C for 15-20 minutes, then the beads were filtered on a sintered glass funnel and washed with ice-cold 0.1 M HCl. The beads were immediately washed with 0.1 M sulphamic acid and then ice-cold water, and resuspended in 0.2 M NaHCO₃, pH 6.0 (10 volumes). The percent of EDC binding to the sepharose beads was between 90-98%. 1.0 ml of gel was loaded on an Econo column (Biorad, Richmond, CA) and washed with about 100 bead volumes of PBS. After the column was packed, conditioned media, derived from SK-Br-3 human breast cancer cells, were run through the beads by gravity (flow rate 30 ml/hr). The column was then washed with 5 volumes of PBS and eluted stepwise with 1.0 M Citric Acid at different pHs (from 4.0 to 2.0). Usually 10 bed volumes of each pH solution were employed. All fractions were desalted on PD10 columns (Pharmacia, Piscataway, NJ) before testing their biological activity. Aliquots from the input media and from the fractions containing activity were analyzed by a 10 % SDS-PAGE, followed by silver staining. Lane 3 shows unconcentrated conditioned media from SK-Br-3 cells. Lane 2 represents the elution at pH 3.0, lane 1 represents the elution at pH 3.5. Sizes are shown in kilodaltons.

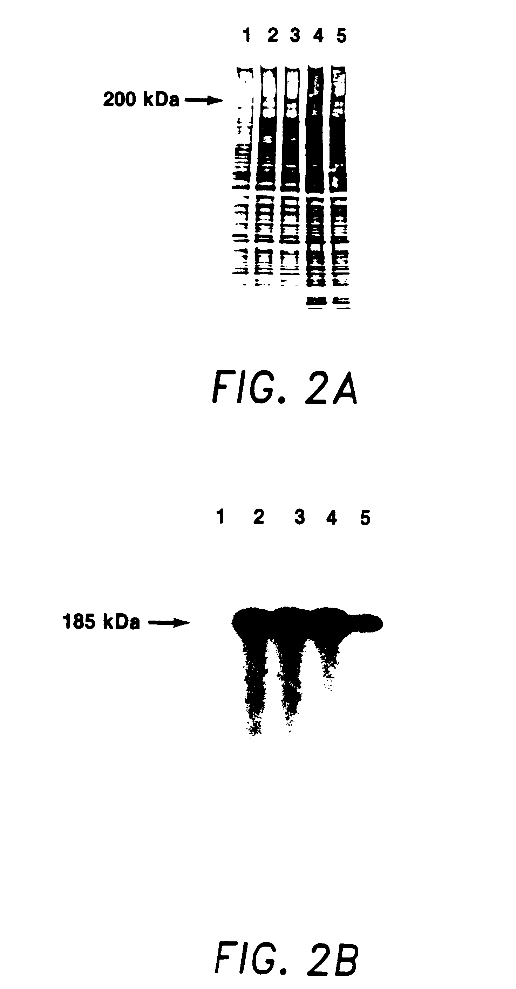

Figure 2 shows detection of phosphorylated proteins from cells incubated in the presence of gp30 or p75. Control media did not contain these ligand molecules.

Figure 2A: MDA MB-453 cells were growth to 90% confluence in 24-well plate (Costar). Cells were treated for 20 minutes at 37°C with control media containing 20 mM Hepes, pH 7.4 (lane 1), control media containing 5 ng/ml of gp30 (lanes 2), control media containing 4 ng/ml p75 (lane 3), control media containing 8 ng/ml p75 (lane 4), and control media containing 2 ng/ml p75 (lane 5). The media was removed and cells were lysed in 100 µl of sample buffer containing 1% SDS, 0.1 % β-mercaptoethanol, 0.15 M Tris-HCl (pH 6.8), 10% glycerol, 0.02% bromophenol-blue, 1 mM EDTA, 2 mM phenylmethyl sulphonyl fluoride (PMSF) and 24 mM leupeptin. After 5 minutes at 95 °C, 50 µg of protein were loaded in a 7.5 % SDS-PAGE. Proteins were then transferred to a nitrocellulose membrane for immunoblotting (Heofer Scientific Instruments, California) by electrophoresis in a modified method of Towbin et al., PNAS, 76, 4350 (1987), using a Hoeffer electrophoretic transfer unit (Hoeffer, Model number TE 22). Electrophoretic transfer was carried out at room temperature for one hour at 125 mA in a buffer containing 25 mM glycine, 129 mM Tris (pH 8.3) and 20% methanol. Following transfer, the filter was blocked with 5% BSA in Tris-Buffered Saline containing 0.5% Tween 20. An anti-phosphotyrosine antibody (Amersham) reacted with the immobilized proteins in 5% BSA (Sigma RIA Grade). Immune-complexes wee detected by a goat anti-mouse antibody conjugated to alkaline phosphatase. The blots were then incubated with a color development substrate solution containing NBT and BCIP (Promega).

Figure 2B: MDA MB-453 cells were growth to 80% confluence in 24-well plates (Costar). The cells were washed then twice with PO₄ free culture media [PO₄] free MEM (GIBCO), with 10% PO₄ free dialyzed fetal calf serum (FCS) and 2 mM glutamine. The cells were then grown in 3 ml/well of PO₄ free culture media for 24 hrs, subsequently the column of medium was adjusted to 1.0 ml/well and .5 mCi of [³²Pi]/well was added and the cells were incubated for 6 hrs. Cells were treated for 20 minutes at 37°C with control media (lanes 1), control media containing 2.0 ng/ml of gp30 (lanes 2), control media containing 4.0 ng/ml p75 (lane 3), control media containing 8.0 ng/ml p75 (lane 4), and control media containing 2.0 ng/ml p75 (lane 5). Following the incubation, the culture dishes were placed over ice-bath and the cells were washed twice with PBS at 4 °C for 10 minutes with 200 µl/well lysis buffer (50 mM Hepes, 150 mM NaCl, 1.0% Triton x-100, 1 mM EGTA, 5 mM EDTA, 10 % GLYCEROL, 02. mM sodium orthovanadate, 0.5 mM PMSF, 20 µg/ml leupeptin, and 5 mM ATP, final pH7.5). The cell lysate was centrifuged at 10,000 g for 1 minute at 4 °C. The supernatant was incubated with 10 µl of normal rabbit serum (NRS) for 1 hr at 4 °C and the non specific complexes were clarified using protein A-sepharose (Sigma). The supernatant was incubated with a polyclonal antibody against the *erb*B-2 C-Terminal sequence Hudziak et al., Proc. Natl. Acad. Sci. USA, 84:7159 (1987). The specific complexes were precipitated with protein A-sepharose (Sigma) and the pellets were washed three times with lysis buffer and the pellet was then resuspended in 50 µl sample buffer (50 mM Tris- HCl (pH 6.8), 2% SDS, 10% Glycerol, 0.1% bromophenol blue and 5% beta-mercaptoethanol). After 5 minutes at 95 °C, the samples were loaded in a 7.5 % SDS-PAGE.

Figure 2C: MDA MB-468 cells were grown to 80% confluence in 24-well plate (Costar). The cells were washed twice with PO₄ free culture media [PO₄] free MEM (GIBCO), with 10% PO₄ free dialyzed fetal calf serum (FCS) and 2 mM glutamine. The cells were then grown in 3 ml/well of PO₄ free culture media for 24 hrs. Subsequently the volume of medium was adjusted to 1.0 ml/well and 0.5 mCi of [³²Pi]/well was added and the cells were incubated for 6 hrs. Cells were treated for 20 minutes at 37 °C with control media (lane 2), control media containing 10.0 ng/ml of p75 (lane 1), control media containing 4.0 ng/ml TGFα (lane 3), control media containing 4.0 ng/ml EGF (lane 4), and control media containing 2.0 ng/ml gp30 (lane 5). Following the incubation the culture dishes were placed over an ice-bath and the cells were washed twice with PBS at 4 °C. Then, cells were lysed at 4 °C for 10 minutes with 200 µl/well lysis buffer (50 mM Hepes, 150 mM NaCl, 1.0% Triton x-100, 1 mM EGTA, 5 mM EDTA, 10% glycerol, 0.2 mM sodium orthovanadate, 0.5 mM PMSF, 20 µg/ml leupeptin, and 5 mM ATP, final pH 7.5). The cell lysate was centrifuged at 10,000 g for 1 minute at 4 °C. The supernatant was incubated with 10 µl of normal rabbit serum (NRS) for 1 hr at 4 °C and the non specific complexes were clarified using protein A-sepharose (Sigma). The supernatant was incubated with a polyclonal antibody against the EGFR (Oncogene Science, N.Y). The specific complexes were precipitated with protein A-sepharose (Sigma) and the pellets were washed three times with lysis buffer and the pellet was then resuspended in 50 µl sample buffer (50 mM Tris-HCl (pH 6.8), 2% SDS, 10% Glycerol, 0.1% bromophenol blue and 5% beta-mercaptoethanol). After 5 minutes at 95 °C, the samples were loaded in a 7.5 % SDS-PAGE.

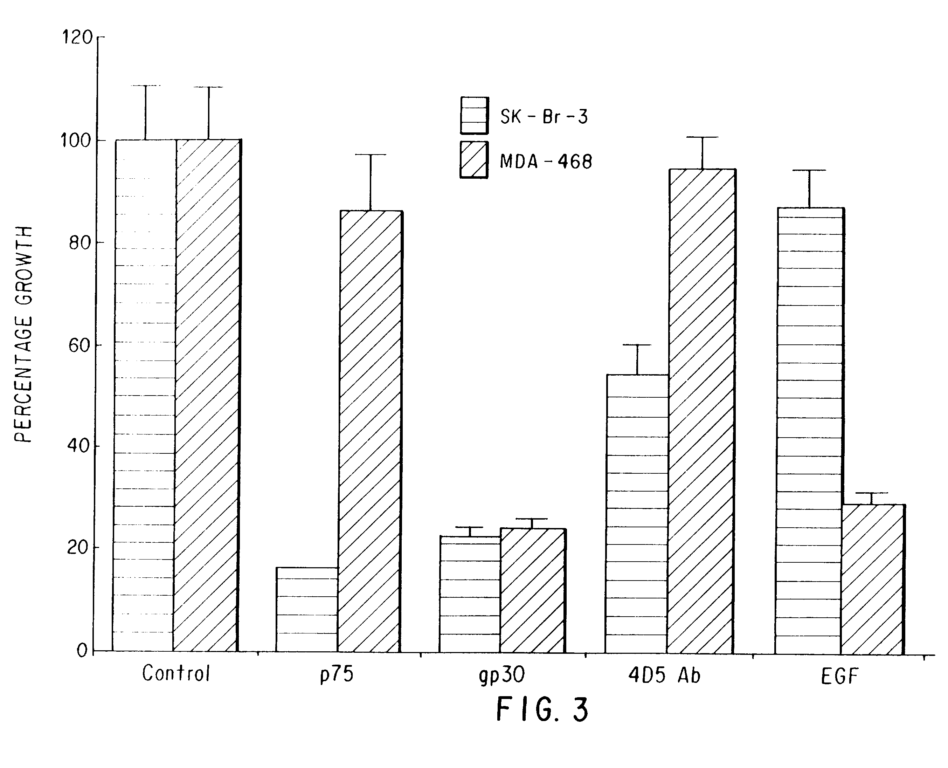

Figure 3 depicts the effect of p75 on the growth of human breast cancer cells. SK-Br-3 and MDA 468 cells were plated (30,000 cells/well) in 24 well plates in IMEM (Biofluids) supplemented with 5% FCS. After 24 hrs the media was removed ad replaced with control serum free media containing fibronectin, transferrin, hepes, glutamine, trace elements, and BSA (SFM+), or SFM+ with the addition of purified p75 (4 ng/ml), purified gp30 (2.0 ng/ml) or with EGF (10 ng/ml). Cells were grown to 90 % confluence of control and counted. Each group was assayed in triplicate. Results are shown as growth relative to control. The experiments were performed three times and the results were reproducible.

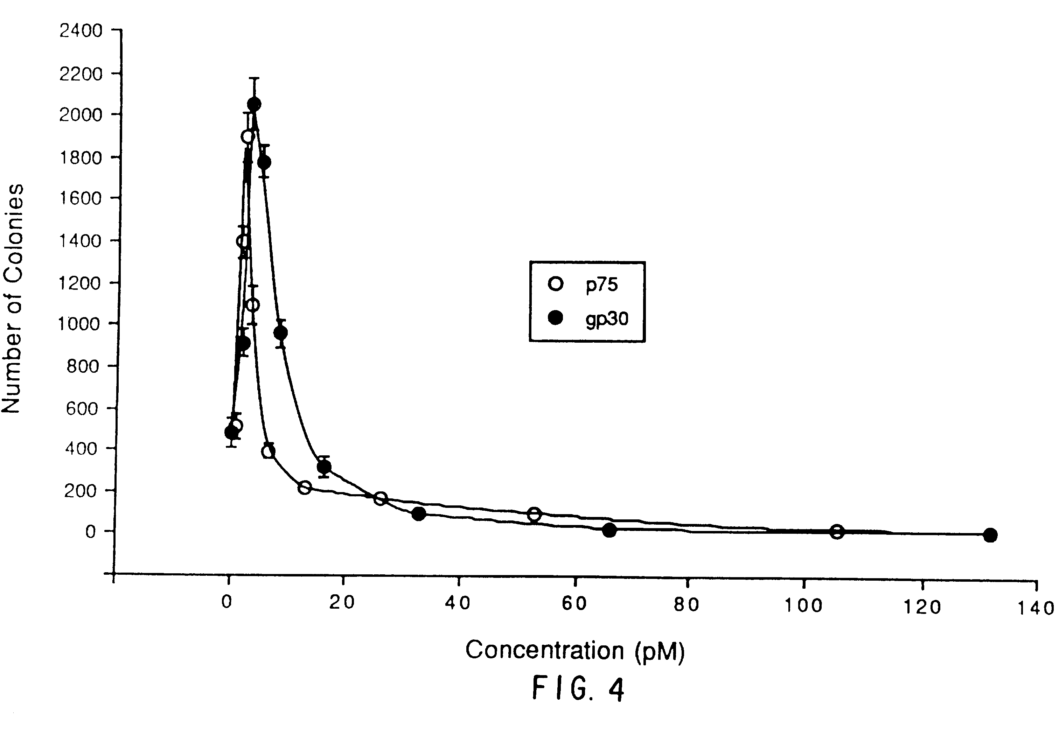

Figure 4 shows the effect of p75 on the soft agar colony formation of human breast cancer cells. SK-Br-3 cells were plated in 35 mm tissue culture dishes (Costar, Cambridge, MA). A bottom layer of 1.0 ml IMEM (Biofluids) containing 0.6% agar and 10 % fetal calf serum (FCS) was prepared. After the bottom layer was solidified, 10,000 cells per dish were added in a 0.8 ml top layer containing the cells, 0.4 % Bacto agar (Difco, Detroit, MI) with 10 % FCS alone and with increasing concentrations of p75 (0-132 pM), and gp30 (0-330 pM). All samples were run in triplicate and experiments were carried out in FCS that had been tested for optimal cloning efficiency. Cells were incubated 7-9 days at 37 °C in 5 % CO₂. Colonies larger than 60 µm were counted in a colony counter. The experiments were performed three times and the results were reproducible.

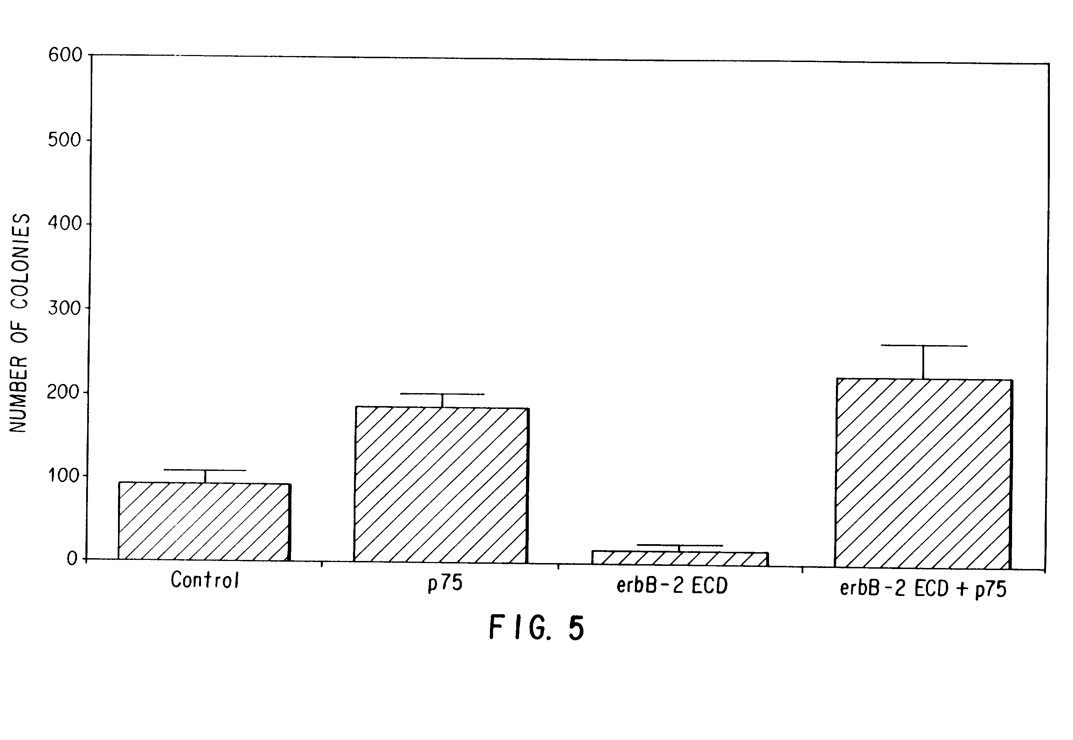

Figure 5 shows the effect of soluble p185^{erbB-2} extracellular domain on the soft agar colony formation of human breast cancer cells. SK-Br-3 cells were plated in 35 mm tissue culture dishes (Costar, Cambridge, MA). A bottom layer of 1.0 ml IMEM (Biofluids) containing 0.6% agar and 10 % FCS was prepared after the bottom layer was solidified. The indicator cell, 10,000 cells per dish were added in a 0.8 ml top layer containing the sample, 0.4% Bacto agar (Difco, Detroit, MI) and 10% FCS. The samples were p75 (0.15 ng/ml), soluble recombinant ECD (12µg/ml) and p75 in the presence of EDC. All samples were run in triplicates and experiments were carried out in FCS that had been tested for optimal cloning efficiency. Cells were incubated 7-9 days at 37 °C in 5% CO₂. Colonies larger than 60 µm were counted in a colony counter. The experiments were performed three times and the results were reproducible.

### Description of the Preferred Embodiments

In the description that follows, a number of terms used in the field of ligand-growth factor receptor interactions and recombinant DNA technology are extensively utilized. In order to provide a clearer and consistent understanding of the specification and claims, including the scope to be given such terms, the following definitions are provided.

Mutant. As used herein, the term "mutant" is meant to include derivatives of an erbB-2 ligand in which the amino acid sequence of the protein has been modified in a manner resulting from addition, substitution, insertion or deletion of one or more amino acids in or from the wild type protein. By a "biologically active mutant" of a erbB-2 ligand is meant a mutant of the ligand which retains all or some of the biological activity possessed by the ligand, particularly the receptor binding activity. Mutation may also be used as a general term to denote the modification of any DNA or RNA sequence by addition, substitution, insertion or deletion of one or more nucleotides within that sequence.

Functional Derivative. By a "functional derivative" of the erbB-2 ligand of the invention is meant a ligand that possesses a biological activity which is substantially similar to the ligand from which the derivative is derived. By "substantially similar" is meant a biological activity which is qualitatively similar but quantitatively different from an activity possessed by a normal erbB-2 ligand. By the phrase "a biological activity which is qualitatively similar" is meant a ligand which more or less retains the biological activity of the natural erbB-2 ligand. For example, a functional derivative of the erbB-2 ligand retains the p185^{erbB-2} receptor binding activity. The term "functional derivative" is intended to include biologically active "mutants," "fragments," and "variants," of the erbB-2 ligand.

Fragment. A "fragment" of the erbB-2 ligand is meant to refer to an protein molecule which contains a portion of the complete amino acid sequence of the wild type ligand. By a "biologically active fragment" of a ligand is meant a fragment of the erbB-2 ligand which retains all or some of the biological activity possessed by the ligand. For example, if the fragment retains some or all of the receptor binding activity, then such fragment is said to be a biologically active fragment of erbB-2 ligand.

Variant. A "variant" of the erbB-2 ligand is meant to refer to a ligand substantially similar in structure and biological activity to either the native erbB-2 ligand or to a fragment thereof, but not identical to such molecule or fragment thereof. A variant is not necessarily derived from the native molecule and may be obtained from any of a variety of similar or different cell lines. The term "variant" is also intended to include genetic alleles. Thus, provided that two erbB-2 ligands possess a similar structure and biological activity, they are considered variants as that term is used herein even if the composition or secondary, tertiary, or quaternary structure of one of the ligands is not identical to that found in the other.

erbB-2 ligand. The term "erbB-2 ligand" is meant to refer to a protein molecule which is capable of binding to an erbB-2 transmembrane protein (p185^{erbB-2}) but fails to bind to the epidermal growth factor receptor. As used herein, the term "erbB-2 ligand" is meant to include any functional derivative of the erbB-2 ligand of the present invention. The erbB-2 ligands of the present invention may bind other protooncogene encoded transmembrane proteins such as c-kit, neu, ros, etc., and thus the term "erbB-2 ligand" is not limited to protein molecules which only bind the erbB-2 transmembrane receptor. Binding of the erbB-2 ligand molecules of the present invention may induce cellular responses of cells which express such other protooncogenes and thus may be used to treat and diagnose patients that have malignant cells which express these other protooncogenes.

### A. Ligands of the erbB-2 Transmembrane Protein

Lupu et al., Science, 249: 1552-1555 (1990) reported the identification and purification of a 30 kilodalton (kDa) growth factor secreted by MDA MB-231 human breast cancer cells. This glycoprotein (gp30) was purified to apparent homogeneity by sequential low affinity heparin-sepharose chromatography and by reversed phase chromatography.

The gp30 glycoprotein binds to epidermal growth factor receptor (EGFR) and has TGFα-related properties. In addition, purified gp30 stimulated phosphorylation of p185^{erbB-2} in cells that overexpress erbB-2, in contrast with TGFa and EGF which do not interact with p185^{erbB-2}.

Surprisingly, gp30 inhibited cell growth in all cells that overexpressed erbB-2 (Lupu et al., Science, 249:1552 (1990)). A monoclonal antibody (4D5) against the extracellular domain of p185^{erbB-2} (Hudziak et al., Molec. Cell. Biol. 9:1165 (1989)) was able to compete with gp30 for binding to p185^{erbB-2}, indicating that the gp30 ligand recognized and bound to the 4D5 binding site.

The 185 kd transmembrane glycoprotein known as p185^{erbB-2} is thought to be a transmembrane protein which functions as a growth factor receptor and is encoded by a protooncogene. The erbB-2 expression is amplified in many adenocarcinomas and, in particular, is amplified or overexpressed in nearly 30% of human breast cancers (Magurie et al., Seminars in Oncology, 16(2):148-155 (1989)). Patients with cancer cells which overexpress erbB-2 are known to have much shorter disease-free periods and poorer overall survival than cancer patients that do not show erbB-2 overexpression. Consequently, it is important to distinguish between malignancies which exhibit erbB-2 overexpression from those which do not. Diagnosis of erbB-2 associated cancers thus provide the clinician with a way to pre-select an effective therapy for treating particular types of cancer.

The erbB-2 ligand of the present invention is extremely important because of the specificity for p185^{erbB-2}. Surprisingly, the erbB-2 ligand does not recognize or bind to EGFR, a highly homologous receptor to p185^{erbB-2}. This characteristic allows the design of diagnostic and therapeutic agents specifically directed against carcinoma cells which overexpress erbB-2.

The erbB-2 ligand of the present invention is a 75 kilodalton protein (p75), although the invention is intended to include any functional derivatives of this factor. Substantially purified p75 ligand competes with 4D5 (Hudziak et al., Mol. Cell. Biol., (9:1165 (1989)) and MOI93 antibodies for p185^{erbB-2} binding and induces phosphorylation of p185^{erbB-2}. In cell growth assays, cell proliferation and colony formation of cell lines overexpressing erbB-2 were inhibited with p75. Furthermore, p75 can reverse the antiproliferative effect of soluble erbB-2 extracellular domain.

### B. Identification of erbB-2 Ligands

Identification of erbB-2 ligands of the present invention can be accomplished by using a radioreceptor assay to screen conditioned media from a number of cells. Any cell type may be used in a screen to isolate ligand producing cells. Preferably, erbB-2 overexpressing cells are used.

The radioreceptor assay, according to the present invention, utilizes a labeled antibody which binds to the extracellular domain of p185^{erbB-2}. Antibodies directed against the erbB-2 receptor extracellular domain are well known. The preferred antibodies for identifying erbB-2 ligands of the present invention are 4D5 (Hudziak et al., Mol. Cell. Biol., 9:1165 (1989)) and MOI93 (Kasprzyk et al., American Assc. Cancer Res. Abstracts (1990)). 4D5 and MOI93 may be obtained from Genentech, CA and Molecular Oncology Inc., MD, respectively.

The antibodies 4D5 and MOI93 recognize and bind to the same binding site of the extracellular domain of p185^{erbB-2} such that the erbB-2 ligand of the present invention is inhibited from binding the receptor in the presence of these antibodies. Thus, these antibodies can be used in competitive binding assays to identify cell lines that produce the erbB-2 ligand of the invention. One of skill in the art will appreciate that other antibodies which recognize different binding sites or epitopes on the extracellular domain can be generated by well known techniques to identify a number of ligands not previously described. Thus, use of different antibodies which bind distinct locations on the extracellular domain of p185^{erbB-2} may provide for the isolation of unique ligands.

In the assay of the present invention, conditioned media is prepared according to commonly employed procedures. For instance, media from a cell culture is cleared from cells and concentrated 100 fold in an Amicon ultrafiltration unit (Yarden et al., Proc. Natl. Acad. Sci., 86:3179-3183 (1989); Lupu et al., (submitted for publication JBC); and Bates et al., Cancer Res. 46:1707-1713 (1986).

Competitive binding of the labeled antibody to p185^{erbB-2} in the presence of conditioned media provides a method for detecting cells which produce ligands. In this manner, the ligand in the conditioned media will compete with the labeled antibody for binding to the p185^{erbB-2} protein. Monitoring the amount of label bound to erbB-2 protein is determinative of the presence of ligand. For example, decrease in label attached to the erbB-2 receptor indicates the presence of ligand.

### C. Purification of erbB-2 Ligand

In accordance with this invention, erbB-2 ligand can be isolated from a cell producing the ligand. Any cell that produces the ligand may be used as a starting material according to the methods described in this invention. The cell type will typically be a cell which overexpresses erbB-2 receptor. Preferrably, SK-Br-3 is used to isolate the 75 kDa erbB-2 ligand of the present invention. This stain is well known to those of skill in the art and is deposited with the American Type Culture Collection, Rockville, Maryland, USA (accession number ATCC, HTB 30). However, any cell which is found to contain the erbB-2 ligand or functional derivatives thereof can be used to isolate and purify such a factor from the cell and/or its culture medium. The ligands of the present invention can, for example, be isolated from a host cell which expresses a recombinant ligand.

The ligand of the present invention is likely to be excreated from the cell. Accordingly, the ligand will normally be purified from the culture media. However, cellular extracts may serve as a source from which to purify the ligand of the present invention.

Typically, the cells producing the desired ligand are grown in media conducive to cell growth. The cells are removed and the desired ligand is purified from the media.

The ligands of the present invention can be extracted and purified from the culture media or cell by using known protein purification techniques commonly employed, such as extraction, precipitation, ion exchanges chromatography, affinity chromatography, gel filtration and the like. The most preferred method to isolate the erbB-2 ligand of the present invention is by affinity chromatography using the erbB-2 receptor extracellular domain bound to a column matrix.

As will be apparent to those of skill in the art, extracellular domain obtained from a natural host producing the protein can be used to purify the ligand of the present invention. For example, SK-Br-3 cells have been used to purify the extracellular domain of p185^{erbB-2} (Alper et al., Cell Growth and Differentiation 1:591-599 (1990)). Alternatively, purified recombinant extracellular domain of p185^{erbB-2} can be used in affinity chromatography to obtain the erbB-2 ligand of the present invention. It will be appreciated that the whole p185^{erbB-2} receptor protein or portions of such a protein may be used to purify the erbB-2 ligand according to the present invention, provided that the protein bound to the column matrix contains the desired erbB-2 ligand binding site of the extracellular domain. Yamamoto et al., Nature 319:230-234 (1986) describes cloning and expression of the full length p185^{erbB-2} gene. A plasmid containing the erbB-2 receptor gene can be obtained from the American Type Culture Collection, Rockville, MD. (Accession No. ATCC 57584).

Using an affinity chromatography purification procedure, erbB-2 ligand was substantially purified from the cellular media. As used herein, the term "substantially pure" or "substantially purified" is meant to describe a ligand which is substantially free of any compound normally associated with the protein in its natural state, i.e., substantially free of contaminating protein and carbohydrate components. The term is further meant to describe a ligand of the present invention which is homogeneous by one or more purity or homogeneity characteristics used by those of skill in the art. For example, substantially pure ligand proteins will show constant and reproducible characteristics within standard experimental deviations for parameters such as the following: molecular weight, chromatographic techniques, and such other parameters. The term, however, is not meant to exclude artificial or synthetic mixtures of the erbB-2 ligand with other compounds. The term is also not meant to exclude the presence of minor impurities which do not interfere with the biological activity of the enzyme, and which may be present, for example, due to incomplete purification.

### D. Cloning erbB-2 Ligand Genes

Any of a variety of procedures may be used to clone the erbB-2 ligand genes of the present invention. One such method entails analyzing a shuttle vector library of DNA inserts (derived from a cell which expresses the erbB-2 ligand) for the presence of an insert which contains the ligand gene. Such an analysis may be conducted by transfecting cells with the vector and then assaying for expression of the ligand binding activity. The preferred method for cloning these genes entails determining the amino acid sequence of the erbB-2 ligand protein. To accomplish this task the desired ligand protein may be purified and analyzed by automated sequencers. Alternatively, each protein may be fragmented as with cyanogen bromide, or with proteases such as papain, chymotrypsin or trypsin (Oike, Y. et al., J. Biol. Chem. 257:9751-9758 (1982); Liu, C. et al., Int. J. Pept. Protein Res. 21:209-215 (1983)). Although it is possible to determine the entire amino acid sequence of these proteins, it is preferable to determine the sequence of peptide fragments of these molecules.

Once one or more suitable peptide fragments have been sequenced, the DNA sequences capable of encoding them are examined. Because the genetic code is degenerate, more than one codon may be used to encode a particular amino acid (Watson, J.D., In: Molecular Biology of the Gene, 3rd Ed., W.A. Benjamin, Inc., Menlo Park, CA (1977), pp. 356-357). The peptide fragments are analyzed to identify sequences of amino acids which may be encoded by oligonucleotides having the lowest degree of degeneracy. This is preferably accomplished by identifying sequences that contain amino acids which are encoded by only a single codon. Although occasionally such amino acid sequences may be encoded by only a single oligonucleotide, frequently the amino acid sequence can be encoded by any of a set of similar oligonucleotides. Importantly, whereas all of the members of the set contain oligonucleotides which are capable of encoding the peptide fragment and, thus, potentially contain the same nucleotide sequence as the gene which encodes the peptide fragment, only one member of the set contains a nucleotide sequence that is identical to the nucleotide sequence of this gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

In a manner exactly analogous to that described above, one may employ an oligonucleotide (or set of oligonucleotides) which have a nucleotide sequence that is complementary to the oligonucleotide sequence or set of sequences that is capable of encoding the peptide fragment.

A suitable oligonucleotide, or set of oligonucleotides which is capable of encoding a fragment of the desired erbB-2 ligand gene (or which is complementary to such an oligonucleotide, or set of oligonucleotides) is identified (using the above-described procedure), synthesized, and hybridized, by means well known in the art, against a DNA or, a cDNA preparation depending upon the source of the gene. Typically, isolation of eukaryotic genes is done by screening a cDNA library, while a DNA library is used to isolate prokaryotic genes. Techniques of nucleic acid hybridization are disclosed by Maniatis, T. et al., In: Molecular Cloning, a Laboratory Manual, Second Edition, Coldspring Harbor, NY (1989), and by Haymes, B.D. et al., In: Nucleic Acid Hybrization, a Practical Approach, IRL Press, Washington, DC (1985).
The source of DNA or cDNA used will preferably have been enriched for the desired sequences. Such enrichment can most easily be obtained from cDNA obtained by extracting RNA from cells cultured under conditions which induce erbB-2 ligand synthesis.

Techniques such as, or similar to, those described above have successfully enabled the cloning of genes for human transforming growth factor-alpha (Derynck et al., Cell 38 (1):287-298 (1984)), chicken epidermal growth factor receptor (Lax et al., Mol. Cell. Biol. 8 (5) :1970-1978 (1988)), human aldehyde dehydrogenases (Hsu, L.C. et al., Proc. Natl. Acad. Sci. USA 82:3771-3775 (1985)), fibronectin (Suzuki, S. et al., Eur. Mol. Biol. Organ. J. 4:2519-2524 (1985)), the human estrogen receptor gene (Walter, P. et al., Proc. Natl. Acad. Sci. USA 82:7889-7893 (1985)), tissue-type plasminogen activator (Pennica, D. et al., Nature 301:214-221 (1983)) and human term placental alkaline phosphatase complementary DNA (Kam, W. et al., Proc. Natl. Acad. Sci. USA 82:8715-8719 (1985)).

In a alternative way of cloning the erbB-2 ligand genes of the present invention, a library of expression vectors is prepared by cloning DNA or cDNA, from a cell capable of expressing such a ligand into an expression vector. The library is then screened for members capable of expressing a protein which binds to an anti-ligand molecule (antibody or blocking peptide) and which has a nucleotide sequence that is capable of encoding polypeptides that have the same amino acid sequence as the erbB-2 ligand protein of the present invention, or fragments or variants thereof.

### E. Expression of erbB-2 Ligand Genes

DNA molecules comprising an erbB-2 ligand gene or at least portions of this gene can be operably linked into an expression vector and introduced into a host cell to enable the expression of the ligand by that cell. Two DNA sequences (such as a promoter region sequence and a desired ligand protein encoding sequence) are said to be operably linked if the nature of the linkage between the two DNA sequences does not (1) result in the introduction of a frame-shift mutation, (2) interfere with the ability of the promoter region sequence to direct the transcription of the desired protein encoding gene sequence, or (3) interfere with the ability of the desired protein gene sequence to be transcribed by the promoter region sequence.

A DNA sequence encoding an erbB-2 ligand protein may be recombined with vector DNA in accordance with conventional techniques. The present invention encompasses the expression of the desired fusion proteins in either prokaryotic or eukaryotic cells. Eukaryotic hosts include yeast (especially Saccharomyces), fungi (especially Aspergillus), mammalian cells (such as, for example, human or primate cells) either in vivo, or in tissue culture.

Yeast and mammalian cells provide substantial advantages in that they can also carry out post-translational peptide modifications including glycosylation. A number of recombinant DNA strategies exist which utilize strong promoter sequences and high copy number of plasmids which can be utilized for production of the desired proteins in these hosts.

Yeast recognize leader sequences on cloned mammalian gene products and secrete peptides bearing leader sequences (i.e., pre-peptides). Mammalian cells provide post-translational modifications to protein molecules including correct folding or glycosylation at correct sites.

Mammalian cells which may be useful as hosts include cells of fibroblast origin such as VERO or CHO-K1, and their derivatives. For a mammalian host, several possible vector systems are available for the expression of the desired fusion protein. A wide variety of transcriptional and translational regulatory sequences may be employed, depending upon the nature of the host. The transcriptional and translational regulatory signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

The expression of the desired fusion protein in eukaryotic hosts requires the use of eukaryotic regulatory regions. Such regions will, in general, include a promoter region sufficient to direct the initiation of RNA synthesis. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); the yeast gal4 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)).

As is widely known, translation of eukaryotic mRNA is initiated at the codon which encodes the first methionine. For this reason, it is preferable to ensure that the linkage between a eukaryotic promoter and a DNA sequence which encodes the desired fusion protein does not contain any intervening codons which are capable of encoding a methionine (i.e., AUG). The presence of such codons results either in the formation of a fusion protein (if the AUG codon is in the same reading frame as the desired fusion protein encoding DNA sequence) or a frame-shift mutation (if the AUG codon is not in the same reading frame as the desired fusion protein encoding sequence).

The expression of the erbB-2 ligand protein can also be accomplished in procaryotic cells. Preferred prokaryotic hosts include bacteria such as E. coli, Bacillus, Streptomyces, Pseudomonas, Salmonella, Serratia, etc. Bacterial hosts of particular interest include E. coli K12, and other enterobacteria (such as Salmonella typhimurium or Serratia marcescens), and various Pseudomonas species. The prokaryotic host must be compatible with the replicon and control sequences in the expression plasmid.

To express the desired ligand protein in a prokaryotic cell (such as, for example, E. coli, B. subtilis, Pseudomonas, Streptomyces, etc.), it is necessary to operably link the desired ligand protein encoding sequence to a functional prokaryotic promoter. Such promoters may be either constitutive or, more preferably, regulatable (i.e., inducible or derepressible). Examples of constitutive promoters include the int promoter of bacteriophage λ, and the bla promoter of the b-lactamase gene of pBR322, etc. Examples of inducible prokaryotic promoters include the major right and left promoters of bacteriophage λ (P_{L} and P_{R}), the trp, recA, lacZ, lacI, gal, and tac promoters of E. coli, the a-amylase (Ulmanen, I., et al., J. Bacteriol. 162:176-182 (1985)), the s-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream from the gene-encoding sequence. Such ribosome binding sites are disclosed, for example, by Gold, L., et al. (Ann. Rev. Microbiol. 35:365-404 (1981)).

The desired protein encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the desired ligand molecule may occur through the transient expression of the introduced sequence. Alternatively, permanent expression may occur through the integration of the introduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may complement an auxotrophy in the host (such as leu2, or ura3, which are common yeast auxotrophic markers), biocide resistance, e.g., antibiotics, or heavy metals, such as copper, or the like. The selectable marker gene can either be directly linked to the DNA gene sequences to be expressed, or introduced into the same cell by co-transfection.

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Any of a series of yeast gene expression systems can be utilized. Examples of such expression vectors include the yeast 2-micron circle, the expression plasmids YEP13, YCP and YRP, etc., or their derivatives. Such plasmids are well known in the art (Botstein, D., et al., Miami Wntr. Syrup. 19:265-274 (1982); Broach, J.R., In: The Molecular Biology of the Yeast Saccharomyces: Life Cycle and Inheritance, Cold Spring Harbor Laboratory, Cold Spring Harbor, NY, p 445-470 (1981); Broach, J.R., Cell 28:203-204 (1982)).

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host chromosome. Cells which have stably integrated the introduced DNA into their chromosomes may be selected by also introducing one or more markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cell. Biol. 3:280 (1983), and others.

Preferred prokaryotic vectors include plasmids such as those capable of replication in E. coli such as, for example, pBR322, ColE1, pSC101, pACYC 184, πVX. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and Streptomyces bacteriophages such as φC31 (Chater, K.F., et al., In: Sixth International Symposium on Actinomycetales Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33:729-742 (1978)).

Once the vector or DNA sequence containing the construct has been prepared for expression, the DNA construct may be introduced (transformed) into an appropriate host. Various techniques may be employed, such as protoplast fusion, calcium phosphate precipitation, electroporation or other conventional techniques. After the fusion, the cells are grown in media and screened for appropriate activities. Expression of the sequence results in the production of the recombinant erbB-2 ligand protein of the present invention.

### F. Purification of Recombinant erbB-2 Ligand

The erbB-2 ligand proteins of this invention can be produced by fermentation of the recombinant host containing the cloned ligand genes. The recombinant host, such as mammalian cells producing the cloned protein, can be grown and harvested according to techniques well known in the art.

The recombinant erbB-2 ligand proteins of the present invention can be extracted and purified from the recombinant host or its culture media by using known protein purification techniques commonly employed, such as extraction, precipitation, ion exchange chromatography, affinity chromatography, gel filtration and the like. Biochemical techniques employed to isolate the erbB-2 ligand proteins of the present invention from SK-Br-3 are of particular interest when purifying these proteins from a recombinant host.

### G. Anti-Ligand Molecules

The present invention concerns anti-ligand molecules which bind covalently or non-covalently to the erbB-2 ligands of the present invention. Without being limited, the anti-ligand molecules of the present invention include antibodies, blocking peptides and any other molecule, compound, chemical, etc. that is capable of covalently or non-covalently binding to the erbB-2 ligand of the present invention.

Blocking peptides, according to the present invention, are "capable of binding" a molecule if they are capable of specifically reacting with or have affinity for the molecule such that the blocking peptide will bind to the molecule. An example of a blocking peptide of the present invention is the extracellular domain of the erbB-2 transmembrane receptor. Typically, peptide fragments of the extracellular domain which bind to the erbB-2 ligand of the present invention may be used, although functional derivatives of such erbB-2 transmembrane receptor may be used. Such derivatives may include, for example, neu, c-kit, met or any transmembrane tyrosine kinases that have a structure reminiscent of growth factor receptors.

The blocking peptides of the present invention may be prepared by a number of well known techniques. Synthetic peptides may be constructed using automated protein synthesizers. Alternatively, the blocking peptides of the invention may be generated through recombinant DNA techniques. For instance, a DNA molecule encoding for the desired peptide may be operably linked to a promoter and other regulatory sequences such that expression of said peptide can be obtained in a transformed host. A number of methods of producing a desired blocking peptide will be readily apparent to one of skill in the art.

It will be understood by those of skill in the art that the blocking peptide of the present invention can be detectably labeled or conjugated with therapeutic agents by standard techniques well known in the art. Examples of detectable labels are described below which may be used to detectably label the blocking peptides of the present invention.

The term "therapeutic agent" as used herein is meant to refer to any molecule, chemical compound, protein etc. which, when introduced in close association to a cell, is capable of killing, destroying, inhibiting the growth or reproduction of, or otherwise interfering in the normal physiology or metabolism of said cell in a manner not conducive to the cell's survival or reproduction. Examples of suitable therapeutic agents include cytotoxic drugs, toxins, isotopes, endocrine therapies and the like. Specific cytotoxic drugs that may be used are Adriamycyn, Cyclophosphamide, 5-Fluorouracil, Methotrexate, Cisplatin, Carboplatin, Vincristine, VP-16, Bleomycin, Mitomycin C, etc. Toxins may include Ricin A, Diftheria, and Pseudomonas. Examples of suitable isotopes include P³², Indium, Yttrium, and Iodine. Examples of suitable endocrine therapy include Diethyl bestrol (DES), Tamoxifen, and LHRH antagonizing drugs.

An antibody is said to be "capable of binding" or "directed against" a molecule if it is capable of specifically reacting with the molecule to thereby bind the molecule to the antibody. The term "epitope" or "binding site" is meant to refer to that portion of an antigen which can be recognized and bound by an antibody. An antigen may have one, or more than one epitope. An "antigen" is capable of inducing an animal to produce antibody capable of binding to an epitope of that antigen. The specific reaction referred to above is meant to indicate that the antigen will react, in a highly selective manner, with its corresponding antibody and not with the multitude of other antibodies which may be evoked by other antigens. The antigen of the present invention can be any ligand identified. Specifically, the p75 erbB-2 ligand can be used to generate anti-p75 ligand antibodies according to the present invention.

The term "antibody" (Ab) or "monoclonal antibody" (Mab) as used herein is meant to include intact molecules as well as fragments thereof (such as, for example, Fab and F(ab')2 fragments) which are capable of binding a hapten or antigen. Fab and F(ab')₂ fragments lack the Fc fragment of intact antibody, clear more rapidly from the circulation, and may have less non-specific tissue binding of an intact antibody (Wahl et al., J. Nucl. Med. 42:316-325 (1983)).

The antibodies used in the present invention may be prepared by any of a variety of methods. For example, cells producing erbB-2 ligand (or fractions, lysates, etc. thereof) can be administered to an animal in order to induce the production of sera contianing polyclonal antibodies that are capable of binding the antigen. Since cells which produce erbB-2 ligand excrete the protein into the cultrue media, the media may be used as a source of the erbB-2 ligand antigen. In a preferred method, a preparation of the erbB-2 ligand of the present invention is prepared and purified to render it substantially free of natural contaminants. Such a preparation is then introduced into an animal in order to produce polyclonal antisera of greater specific activity.

The antibodies of the present invention may be monoclonal or polyclonal antibodies (or hapten binding fragments thereof). Such monoclonal antibodies can be prepared using hybridoma technology (Kohler et al., Nature 256:495 (1975); Kohler et al., Eur. J. Immunol. 6:511 (1976); Kohler et al., Eur. J. Immunol. 6:292 (1976); Hammerling et al., In: Monoclonal Antibodies and T-Cell Hybridomas, Elsevier, N.Y., pp. 563-681 (1981)). In general, such procedures involve immunizing an animal with substantially pure erbB-2 ligand protein.

The splenocytes of the immunized animal are extracted and fused with a suitable myeloma cell line. Any suitable myeloma cell line may be employed in accordance with the present invention; however, a suitable parent myeloma cell line (SP₂O), available from the American Type Culture Collection, Rockville, Maryland, may be used. After fusion, the resulting hybridoma cells are selectively maintained in HAT medium, and then cloned by limiting dilution as described by Wands, J.R., et al. (Gastroenterology 80:225-232 (1981).

The hybridoma cells obtained through such a selection are then assayed to identify clones which secrete antibodies capable of binding to the erbB-2 ligand.

It will be appreciated that Fab and F(ab')₂ and other fragments of the antibody may be used according to the methods disclosed herein for the detection erbB-2 ligand in samples in the same manner as intact antibody. Such fragments are typically produced by proteolytic cleavage, using enzymes such as papain (to produce Fab fragments) or pepsin (to produce F(ab')₂ fragments). Alternatively, hapten-binding fragments can be produced through the applicaiton of recombinant DNA technology or through synthetic chemistry.

Similar to blocking peptides, antibodies can be conjugated to the therapeutic agents. Suitable examples of therapeutic agents which may be conjugated to the to the anti-ligand antibodies of the present invention include, but are not limited to, cytotoxic drugs, toxins, and isotopes. Examples of suitable therapeutic agents are described above.

### H. Assays for Detecting erbB-2 Ligand

The anti-ligand molecules including antibodies, fragments of antibodies, or blocking peptides of the present invention may be used to detect the presence of the erbB-2 ligand. Thus, the antibodies (or fragments thereof) and blocking peptides may be employed in histology and biopsy to detect erbB-2 ligand expression in a patient suffering from breast, liver, ovarian, lung, colon carcinomas and the like. Such detection may be accomplished using any of a variety of assays. For example, by radioactively labeling the antibodies or antibody fragments, it is possible to detect the erbB-2 ligand through the use of radioimmune assays. A good description of a radioimmune assay (RIA) may be found in Laboratory Techniques and Biochemistry in Molecular Biology, by Work, T.S., et al., North Holland Publishing Company, NY (1978), with particular reference to the chapter entitled "An Introduction to Radioimmune Assay and Related Techniques" by Chard, T., incorporated by reference herein. Alternatively, flouresecent, enzyme, or other suitable labels can be employed. Detectably labeled blocking peptides may be used in an analogous manner to detect the erbB-2 ligand.

Alternatively, the detection of erbB-2 ligand may be accomplished by in vivo imaging techniques, in which the labeled antibodies, fragements thereof, or blocking peptides are provided to a patient, and the presence of the breast, ovarian, liver, lung, or colon carcinoma which expresses erbB-2 ligand is detected without the prior removal of any tissue sample. Such in vivo detection procedures have the advantage of being less invasive than other detection methods, and are, moreover, capable of detecting the presence of antigen-expressing cells in tissue which cannot be easily removed from the patient.

In accordance with the above-discussed assays, antibodies, fragments thereof, or blocking peptides may be labeled using any of a variety of labels and methods of labeling. Examples of types of labels which can be used in the present invention include, but are not limited to, enzyme labels, radioisotopic labels, non-radioactive isotopic labels, fluorescent labels, toxin labels, and chemiluminescent labels.

Examples of suitable enzyme labels include malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, acetylcholine esterase, etc.

Examples of suitable radioisotopic labels will be readily apparent to one of skill in the art. Suitable non-radioactive isotopic labels for use in the present invention will also be known to one of ordinary skill in the art.

Examples of suitable fluorescent labels include a fluorescein label, an isothiocyanate label, a rhodamine label, a phycoerythrin label, a phycocyanin label, an allophycocyanin label, an o-phthaldehyde label, a fluorescamine label, etc.

Examples of suitable toxin labels include diphtheria toxin, ricin, and cholera toxin. Examples of chemiluminescent labels include a luminal label, an isoluminal albel, an aromatic acridinium ester label, an imidazole label, an acridiniu salt label, an oxalate ester label, a luciferin label, a luciferase label, an aequorin label, etc.

Those of ordinary skill in the art will know of other suitable lables which may be employed in accordance with the present invention. The binding of these labels to antibodies or fragments thereof can be accomplished using standard techniques commonly known to those of ordinary skill in the art. Typical techniques are described by Kennedy, J.H., et al. (Clin. Chim. Acta 70:1-31 (1976)), and Schurs, A.H.W.M., et al. (Clin. chimp Acta 81:1-40 (1977)). Coupling techniques mentioned in the latter are the glutaraldehyde method, the periodate method, the dimaleimide method, the m-maleimido-benzyl-N-hydroxy-succinimide ester method.

The detection of the antibodies, fragments of antibodies or blocking peptides can be improved through the use of carriers. Well-known carriers include glass, polystyrene, polypropylene, polyethylene, dextran, nylon, amylases, natural and modified celluloses, polyacrylamides, agaroses, and magnetite. The nature of the carrier can be either soluble to some extent or insoluble for the purposes of the present invention. The support material may have virtually any possible structural configuration so long as the coupled molecule is capable of binding to an antigen. Thus, the support configuration may be spherical, as in a bead, or cylindrical, as in the inside surface of a test tube, or the external surface of a rod. Alternatively, the surface may be flat such as a sheet, test strip, etc. Those skilled in the art will note many other suitable carriers for binding antibodies and blocking peptides, or will be able to ascertain the same by use of routine experimentation.

The binding molecules (anti-ligand molecules) of the present invention may also be adapted for utilization in an immunometric assay, also known as a "two-site" or "sandwich" assay. In a typical immunometric assay, a quantity of unlabeled antibody, or fragment of antibody, is bound to a solid support that is insoluble in the fluid being tested (i.e., blood, lymph, liquified, stools, tissue homogenate, etc.) and a quantity of detectably labeled soluble antibody is added to permit detection and/or quantitation of the ternary complex formed between solid-phase antibody, peptide antigen, and labeled antibody. It will be apparent to one of skill that labeled blocking peptide may also be used in place of or combination with the antibody used in the assay according to the invention.

Typical immunometric assays include "forward" assays in which the antibody or blocking peptide bound to the solid phase is first contacted with the sample being tested to extract the antigen from the sample by formation of a binary solid phase antibody-antigen complex or a blocking peptide-antigen complex. After a suitable incubation period, the solid support is washed to remove the residue of the fluid sample, including unreacted antigen, if any, and then contacted with the solution containing an unknown quantity of labeled antibody or labeled blocking peptide (which functions as a "reporter molecule"). After a second incubation period to permit the labeled molecule to complex with the antigen bound to the solid support through the unlabeled antibody or blocking peptide, the solid support is washed a second time to remove the unreacted labeled antibody. This type of forward sandwich assay may be a simple "yes/no" assay to determine whether erbB-2 ligand antigen is present or may be made quantitative by comparing the measure of labeled antibody or labeled blocking peptide with that obtained for a standard sample containing known quantities of antigen. Such "two-site" or "sandwich" assays are described by Wide at pages 199-206 of Radioimune Assay Method, edited by Kirkham and Hunter, E. & S. Livingstone, Edinburgh, 1970.

In another type of "sandwich" assay, which may also be useful to detect the erbB-2 ligand antigen, the so-called "simultaneous" and "reverse" assays are used. A simultaneous assay involves a single incubation step as the antibody or blocking peptide bound to the solid support and labeled antibody or blocking peptide are both added to the sample being tested at the same time. After the incubation is completed, the solid support is washed to remove the residue of fluid sample and uncomplexed labeled antibody or peptide. The presence of labeled antibody or blocking peptide associated with the solid support is then determined as it would be in a conventional "forward" sandwich assay.

In the "reverse" assay, stepwise addition of a solution of labeled antibody or labeled blocking peptide to the fluid sample is followed by the addition of unlabeled antibody or unlabeled blocking peptide bound to a solid support after a suitable incubation period is utilized. After a second incubation, the solid phase is washed in conventiional fashion to free it of the residue of the sample being tested and the solution of unreacted labeled antibody or block peptide. The determination of labeled antibody or labeled blocking peptide associated with a solid support is then determined as in the "simultaneous" and "forward" assays.

As explained above, the immunometric assays for erbB-2 ligand antigen require that the particular binding molecule be labeled with a "reporter molecule." These reporter molecules or labels, as identified above, are conventional and well-known to the art. In the practice of the present invention, enzyme labels are a preferred embodiment. No single enzyme is ideal for use as a label in every conceivable immunometric assay. Instead, one must determine which enzyme is suitable for a particular assay system. Criteria important for the choice of enzymes are turnover number of the pure enzyme (the number of substrate molecules converted to the product per enzyme site per unit of time), purity of the enzyme preparation, sensitivity of detection of its product, ease and speed of detection of the enzyme reaction, absence of interfering factors or of enzyme-like activity in the test fluid, stability of the enzyme and its conjugate, availability and cost of the enzyme and its conjugate, and the like. Included among the enzymes used as preferred labels in the immunometric assays of the present invention are peroxidase, alkaline phosphatase, beta-galactosidase, urease, glucose oxidase, glycoamylase, malate dehydrogenase, and glucose-6-phosphate dehydrogenase.

In addition, the materials for use in the assays of the invention are ideally suited for preparation of a kit. Such a kit may comprise a carrier means being compartmentalized to receive in close confinement one or more container means such as vials, test tubes, and the like. Each of said container means comprises one of the separate elements to be used in the method.

For example, one of said container means may comprise an immuno-absorbent-bound peptide fragement. Such fragment may be bound to a separate solid-phase immunoabsorbent or directly to the inner walls of a container. A second container may comprise detectably labeled antibody or blocking peptide in lyophilized form or in solution.

The carrier may also contain, in addition, a plurality of containers each of which comprises different, predetermined and known amounts of antigen. These latter containers can then be used to prepare a standard curve from which can be interpolated the results obtained from the sample containing the unknown amount of antigen.

### I. Uses of Anti-Ligand Molecules

The anti-ligand molecules of the present invention have a multitude of therapeutic and diagnostic uses. For example, therapeutic uses may involve cancer therapy in a patient suspected of suffering from cancer. Specifically, the anti-ligand molecules of the present invention such as antibodies or blocking peptides may be used to treat patients that have adenocarcinoma cells which produce the erbB-2 ligand and/or overexpress the erbB-2 receptor proteins.

One type of treatment may involve the use of the antibody conjugated to a therapeutic agent. Blocking peptide coupled to a therapeutic agent may be used in an analogous manner. By administering an effective amount of anti-ligand coupled to the therapeutic agent to a patient, the adenocarcinoma cells in the patient which express erbB-2 ligand and/or a erbB-2 receptor can be growth inhibited or killed, thereby providing a treatment for cancer. Normal and malignant cells which overexpress EGFR are not affected by administration of the anti-ligand-therapeutic conjugate agent to a patient. Thus, treatment of a patient with the anti-ligand conjugate may selectively inhibit or destroy erbB-2 overexpressing cancer cells in vivo.

In accordance with the method of cancer treatment of the invention, the conjugated anti-ligand is capable of recognizing and binding to carcinoma cells due to the carcinoma cells association with the erbB-2 ligand. Without being limited, the mechanism of binding to the cancer cell may involve the recognition of erbB-2 ligand located on the cell surface or because of expression and/or secretion of the ligand.

Once the conjugated anti-ligand is bound or in close association with the adenocarcinoma cell by interacting with ligand, the therapeutic agent is capable of inhibiting or killing that cell. In this manner, the therapy of the present invention is selective for a particular target, i.e., cancer cells which are associated with the erbB-2 ligand. Normal cells and other cells not associated with the erbB-2 ligand (cells which do not express or bind erbB-2 ligand) may not, for the most part, be affected by this therapy.

Alternatively, the anti-ligands of the present invention may be used to prevent or inhibit inducement of adenocarcinoma cell proliferation. For example, cancer cells which contain the p185^{erbB-2} receptor are induced to proliferate in the presence of low concentratioons of erbB-2 ligand. Preventing the erbB-2 ligand growth factor from interacting with its receptor may provide a means to treat a cancer patient.

According to the method of inhibiting cellular proliferation of the present invention, the anti-ligand is capable of binding to the erbB-2 ligand. Binding the excreted erbB-2 ligand in vivo forms a ligand-anti-ligand complex and thus may prevent or inhibit the ligand-receptor interaction either sterically or otherwise. Thus, the present invention provides a treatment to prevent or inhibit adenocarcinoma cell proliferation in a patient by administering an effective amount of an anti-ligand to such a patient.

It will be appreciated that a number of other therapeutic uses of the anti-ligands of the present invention may be devised. Such therapies may involve use of other known treatment techniques in combination with the anti-ligands of the invention. The present invention is not meant to be limited to the therapeutic treatment described and are thus only presented by way of illustration.

Furthermore, administration of an effective amount of the anti-ligands of the present invention sufficient to inhibit or kill an adenocarcinoma cell may vary depending upon a number of factors including the type of malignant cell, body weight of the patient, the type of therapeutic agent used and the like. Those of skill in the art will appreciate that the amount necessary to inhibit or kill a particular malignant cell in vitro or in vivo can easily be determined with minimal experimentation.

Diagnostic uses of the anti-ligand molecules of the present invention may include, for example, detection of erbB-2 ligand in a sample obtained from a patient. Such samples may be body tissue, body fluids (such as blood, urine, tear drops, saliva, serum, and cerebrospinal fluid), feces, cellular extracts and the like.

According to the method of detecting erbB-2 ligands, the erbB-2 ligand of the present invention is excreted in vitro into cell culture medium. Another growth factor, TGFα, also secreted in vitro was identified in body fluids of cancer patients. Consequently, the growth factor of the present invention (erbB-2 ligand) may be detected in body fluids, stools, etc. from a cancer patient.

Assaying for the erbB-2 ligand of the invention in a sample obtained from a patient may thus provide for a method for diagnosing cancer. That is, detection of erbB-2 ligand in a sample obtained from a patient indicates the presence of erbB-2 ligand expressing cells in a patient. Furthermore, since the anti-ligand is specific for erbB-2 ligand, the assay may provide information concerning the biology of a patient's tumor. For example, cancer patients with adenocarcinoma cells that overexpress the erbB-2 receptor are known to have a much shorter disease-free period and poorer overall survival than cancer patients that do not show erbB-2 overexpression. Detection of erbB-2 ligand growth factor may thus serve as a prognostic test, allowing the clinician to select a more effective therapy for treating the patient.

### J. Uses of erbB-2 Ligand

The erbB-2 ligand of the present invention may be used both diagnostically and therapeutically. Specifically, the erbB-2 ligand may be used to detect, in a patient, adenocarcinoma cells which overexpress the erbB-2 receptor protein. Treatment of such a patient to growth inhibit or destroy these cells may also be accomplished according to the present invention.

Expression of the erbB-2 protooncogene encoding a 185 kDa transmembrane protein serves as a marker to identify a particular invasive malignant cell type. Since the erbB-2 receptor is a transmembrane protein, its extracellular domain is accessible to interaction with its ligand on the cell surface. Consequently, the ligand of the present invention which binds specifically to p185^{erbB-2} can be utilized to detect cells which express the erbB-2 receptor. Surprisingly, the erbB-2 ligand of the present invention does not react with the EGFR and thus is specific for erbB-2 receptor. Therefore, the erbB-2 ligand of the invention is capable of detecting particular cancer cells in a patient and may not recognize normal cells or malignant cells that fail to overexpress the erbB-2 receptor. This characteristic is important in that early detection of erbB-2 overexpressing malignant cells may indicate prognosis and treatment for the patient.

According to the present invention, diagnosis with the erbB-2 ligand involves the detection of p185^{erbB-2} overexpressing cancer cells in a patient. Detection of such cells in a patient may be accomplished by any of a variety of in vitro assays or in vivo imaging techniques. Examples of these in vitro and in vivo techniques are disclosed in section H in the Description of the Preferred Embodiements. The materials for use in the in vitro assays and in vivo imaging techniques which utilize erbB-2 ligand are also ideally suited for preparation of a kit.

One of skill in the art will recognize that in accordance with these assays, a variety of labels and methods of labeling may be used. Examples of types of labels that can be used in the present invention include, but are not limited to, enzyme labels, radioisotopic labels, non-radioactive isotopic labels, fluorescent labels, toxin labels, and chemiluminescent labels. The binding of these labels to the erbB-2 ligand protein may be accomplished using standard techniques commonly known to those of ordinary skill in the art.

The erbB-2 ligand of the present invention may be used to treat a patient suffering from cancer. Treatment therapies with erbB-2 ligand are specifically targeted against cells which may bind to the erbB-2 ligand of the present invention. In this manner, malignant cells that overexpress the erbB-2 receptor may be growth inhibited or destroyed by the treatment method of the present invention.

It will be appreciated that a number of therapeutic uses of the erbB-2 ligand of this invention may be devised. Thus, the present invention is not meant to be limited to the therapeutic treatments described and thus are only presented by way of illustration.

One aspect of cancer treatment using the erbB-2 ligand of this invention concerns the use of ligand-therapeutic agent conjugates. The erbB-2 ligand conjugates of the invention may bind to the adenocarcinoma cell which overexpress the erbB-2 receptor. Once the erbB-2 ligand conjugate is bound to the cell, the therapeutic agent is capable of killing or inhibiting the growth of that cell.

In this manner, administration of an effective amount of ligand conjugate to a patient serves as a treatment that may destroy or growth inhibit particular types of cancer cells in vivo. Normal and malignant cells which express EGFR are not affected by administration of the ligand-therapeutic agent conjugate of this invention.

A second aspect of treatment using the erbB-2 ligand of the present invention relates to the inhibitory affects of the growth factor. Surprisingly, the erbB-2 ligand of the present invention acts, in sufficient concentrations, as an inhibitor capable of inhibiting or suppressing proliferation of adenocarcinoma cells. Any of a number of cancer cells may be growth inhibited with the erbB-2 ligand of the present invention, provide that the erbB-2 ligand can interact with the cell. Typically, malignant cells which overexpress the erbB-2 receptor are inhibited. Such cells may include, but are not limited to, breast, lung, ovarian, gastric, thyroid, prostate or salivary gland carcinoma cells. Cells not affected by the erbB-2 ligand of the invention include normal cells and malignant cells which do not overexpress the protooncogene coding for the erbB-2 receptor.

In vitro or in vivo inhibition of tumor cells may be accomplished by administration of an effective amount of the erbB-2 ligand of the present invention. One of skill in the art will recognize that the amount sufficient to inhibit cell growth varies depending on the cell type, the body weight of the patient, the type of therapeutic agent used etc. These variables can easily be determined by those skilled in the art with little experimentation.

### Example 1

### Identification of Ligands

A 4D5 radioreceptor assay was used to screen conditioned media derived from different human cell lines for the presence of p185^{erbB-2} binding activity. Conditioned media was collected as described by Bates et al., Cancer Res. 46:1707-1713 (1986). Briefly, media were concentrated 100-fold in an Amicon ultrafiltration cell (YM5 membrane) (Amicon, Danvers, MA). Once clarified and concentrated, the media were stored at -20°C while consecutive collections were made during the following days. The concentrated media were dialyzed using Spectraphore 3 tubing (Spectral Medical Industries, Los Angeles, CA) against 100 volumes of 0.1 M acetic acid over a two day period at 4°C. The material that precipitated during dialysis was removed by centrifugation at 4000rpm for 30 min. at 4°C; proteast inhibitors were added as described by Bates et al., Cancer Res., 46:1707-1703 (1986).

A monoclonal antibody, 6E9, (Fendly B.M. et al., Cancer Res., 50:1550-1558 (1990)) against the extracellular domain of p185^{erbB-2} which does not compete with gp30 for p185^{erbB-2} binding was used as a control, to rule out the possibility that the erbB-2 oncoprotein or the portion of it that is shed from the cells into their growth media might interfere with the 4D5 assay. Shed erbB-2 extracellular domain would compete with the binding of both 4D5 and 6E9 to p185^{erbB-2}, as opposed to a erbB-2 ligand which would compete exclusively with 4D5. Any antibody which is directed against the p185^{erbB-2} extracellular domain can be used as a control antibody in place of 6E9, provided that the control antibody does not interfere with 4D5 or MOI93 binding, i.e., that the control antibody, and the test antibody do not bind the same binding site on the extracellular domain.

Binding assays were performed as described (Lupu et al. , Science, 249:1552 (1990)). Proliferating SK-Br-3 cells were incubated with iodinated anti-erbB-2 antibodies (4D5 or 6E9) in the presence of several concentrations of conditioned media. Media derived from SK-Br-3, MCF-7, HS578T, MDA MB-453, BT-549, MDA MB-468, and MDA MB-157 breast cancer cells, as well as several nonmalignant and transformed breast epithelial cells (Cell lines studied were non-malignant breast epithelial 184 cells, immortalized 184A1N4 cells and oncogene-transformed 184A1N4T (SV-40), 184A1N4H (ras), 184A1N4M (myc), 184A1N4TH (SV-40, myc), and 184A1N4MH (myc, ras) cells were evaluated.) Conditioned media from MDA MB-231 cells were used as a positive control since these cells were known to secrete gp30. Only one of the 15 different media tested, that from SK-Br-3 cells, showed an ability to compete with 4D5 for p185^{erbB-2} binding.

### Example 2

### Characterization and Purification of the erbB-2 Ligand

Since SK-Br-3 cells seemed to secrete a erbB-2 ligand, our next step was to determine whether the putative ligand showed heparin binding, which would be consistent with the secretion of gp30. No p185^{erbB-2} binding activity was detected after media from SK-Br-3 cells was processed by heparin chromatography, suggesting that either gp30 was not present in the media or since SK-Br-3 cells release p185^{erbB-2} extracellular domain (ECD) into the culture media, that ECD might interfere with heparin binding or might be washed off the column bound to the extracellular domain.

Our next step was to develop a purification procedure which would not interfere with p185^{erbB-2} ECD. Recombinant p185^{erbB-2} extracellular domain (ECD) (obtained from Genentech Inc., CA) was coupled to a polyacrylamide-hydrazide sepharose affinity chromatography matrix (Avromeas et al., Scand. J. Immunol., 8:7 (1978)). The molecular weight of this extracellular domain is approximately 94 kilodalton.

The coupling was tested by demonstrating the binding of iodinated 4D5 antibody to the column. Other antibodies such as MOI93 can also be used. Concentrated conditioned media from SK-Br-3 cells were loaded onto the column and elution of the material obtained was performed stepwise with 1.0 M Citric Acid across a pH range from 4.0 to 2.0, to allow the dissociation of the erbB-2 ECD and putative ligand. The fractions were tested for their p185^{erbB-2} binding properties in the 4D5 binding assay.

A single purification yielded an apparent homogeneous polypeptide of 75 kilodaltons at 3.0 - 3.5 elution pH. The homogeneity of the sample was confirmed by analysis on a SDS-PAGE (Laemmli U.K., Nature, 227:680 (1970)) by silver staining (Morissey, J.H., Anal. Biochem., 177:307 (1981)) (Figure 1). This preparation was the source used for further experiments.

To confirm that the 75 kDa polypeptide (p75) obtained from the ECD affinity column at pH 3.0-3.5 elution of SK-Br-3 conditioned media was indeed a ligand for the erbB-2 oncoprotein we used two independent assays. We first confirmed, with the 4D5 radioreceptor assay, that p75 (eluted at pH 3.0-3.5) binds specifically to the erbB-2 receptor in SK-Br-3 cells, while material from other chromatography fractions or flow-through did not show such activity (Table 1). The binding of iodinated 6E9 antibody to p185^{erbB-2} was not altered by p75, suggesting that the eluted material was not p185^{erbB-2} ECD.

**Table 1**

| | %p185^{erbB-2} binding | % EGFR binding |
|---|---|---|
| Control | 100 | 100 |
| Flow-Through | 99 | 96 |
| pH 2 | 99 | 95 |
| pH 2.5 | 91 | 95 |
| pH 3 | 9 | 95 |
| pH 3.5 | 18 | 99 |
| pH 4 | 63 | 98 |

**Table 1. Binding of chromatography fractions from SK-Br-3 cell conditioned medium (p75) to p185**^{**erbB-2**} **and EGFR.** SK-Br-3 and MDA-MB-468 cells were plated (100,000 cells/well) in 24-well plates in 5% FCS IMEM (Biofluids). Binding studies were performed as described (Lupu et al., Sicence 249:1552-1555 (1990)). 50 ml of 100x conditioned media from SK-Br-3 cells were loaded into a p185^{erbB-2} extracellular domain affinity chromatography column. Flow-through and fractions eluted with 1 M citric acid (pH gradient from 4 to 2) were collected. After neutralization (pH 7.4) and desalting with PBS, the fractions were tested for p185^{erbB-2} and EGFR binding activity. Iodinated 4D5 antibody was used to assess p185^{erbB-2} binding in SK-Br-3 cells, and iodinated EGF was used to assess EGFR binding in MDA-MD-468 cells. Results are shown as percent of control (no treatment) binding. Each experiment was performed in triplicate, and the SD were less than 15% in all cases.

Since gp30 had been identified as a ligand common to both the EGFR and p185^{erbB-2}, we also tested the activity of all the eluted fractions from SK-Br-3 conditioned media in a EGFR binding assay using MDA MB-468 cells and iodinated EGF. In this assay EGF and gp30, used as controls, displaced the binding of iodinated EGF in a dose dependent manner. In contrast, none of the eluted fractions derived from SK-Br-3 conditioned media showed activity, indicating that p75 does not bind to the EGFR (Table 1).

Since p75 interacted with the erbB-2 oncogene product, we next explored whether p75 activated p185^{erbB-2}. We studied the ability of p75 to phosphorylate p185^{erbB-2} using MDA MB-453 human breast cancer cells, which overexpress the erbB-2 oncoprotein but do not express detectable levels of EGFR. First we determined that p75 activated tyrosine phosphorylation in MDA MB-453 cells, using an anitphosphotyrosine monoclonal antibody in a Western blot analysis (Towbin et al., Proc. Natl. Acad. Sci. USA, 76:4350 (1979)) (Figure 2A). The specificity of the tyrosine phosphorylation for p185^{erbB-2} was confirmed by labeling MDA MB-453 cells with [³²Pi] and immunoprecipitating the p185^{erbB-2} oncoprotein with a polyclonal antibody that reacts with the C-terminal domain of p185^{erbB-2} but does not show cross-reactivity with EGFR (Hudziak et al., Proc. Natl. Acad. Sci. USA, 84:7159 (1987)) (Figure 2B). No phosphorylation was observed when MDA MB-453 cells were treated with EGF. In control experiments, phosphorylated EGFR was precipitated from MDA MB-468 cells with an anti-EGFR antibody after treatment with gp30, EGF and TGFα, p75, however did not induce phosphorylation of the EGFR in these cells (Figure 2C). These observations supported the hypothesis of an exclusive interaction between p75 and its receptor p185^{erbB-2}.

### Example 3

### Cellular Response to erbB-2 Ligand

We examined the biological effects of p75 in breast cancer cells, using anchorage-dependent and anchorage-independent growth assays. p75 inhibited the cellular proliferation of the overexpressing cells SK-Br-3, BT-474 and MDA MB-453 by 70-80% at a concentration of 4 ng/ml (The concentration of p75 polypeptide was determined by 4D5 binding assay). No inhibition was observed in MDA MB-468 cells, which overexpress the EGFR, or in MCF-7 cells which do not overexpress p185^{erbB-2} or EGFR. gp30, used as control, inhibited the proliferation of SK-Br-3 and MDA MB-468 cells (Figure 3). In an anchorage-independent growth assay, p75 inhibited the soft agar colony formation of SK-Br-3 and MDA MB-453 cells by 60-70%.

Since our initial experiments p75 inhibited the growth of breast cancer cells, we speculated that either p75 was an inhibitory ligand or the inhibitory function of p75 resulted from hyperstimulation of the erbB-2 receptor. We observed that p75 at very low doses 3.3 pM (0.25 ng/ml) had a stimulatory growth effect on SK-Br-3 (Figure 4). The proliferative effects of gp30 at equimolar concentrations were similar to those of p75 (Figure 4). As an additional control, EGF at concentrations from 1-100 nM had no significant effects on the growth of SK-Br-3 cells. The results obtained with p75 argued against the possibility of an inhibitory ligand. Dose-related paradoxical effects of growth factors on cellular proliferation have been reported in the literature.

Low concentrations of gp3C stimulated the growth of SK-Br-3 cells, while high concentrations were growth inhibitory (Lupu et al. , Science, 249:1552 (1990)). EGFR-overexpressing breast cancer cells MDA MB-468, as well as the A431 cancer cells, are growth-inhibited by high doses of EGF but are stimulated by very low doses of EGF (Kawamoto et al. , J. Biol. Chem., 249:7761 (1984); Ennis et al., Molec. Endocr., 3:1830 (1989)). It has also been reported that cells overexpressing the estrogen receptor are growth-inhibited by physiological doses of estrogen (Kushner et al., Molec. Endocr., 4:1465 (1990)).

### Example 4

### Interaction of p75 with p185^{erbB-2} Extracellular Domain

In additional experiments, we explored the interaction of p75 and p185^{erbB-2} soluble extracellular domain. As might have been expected, addition of soluble p185^{erbB-2} ECD to SK-Br-3 cells inhibited their soft agar colony formation. The inhibitory effect of this ECD may be due either to dimerization of the soluble domain with the cellular p185^{erbB-2} receptor, or to binding and neutralization of p75, which is essential for their growth, therefore leading to inhibition of cell proliferation. ECD inhibited colony formation exclusively in those cells overexpressing p185^{erbB-2} (Figure 5). No inhibition was observed in MDA MB-468 and MCF-7 cells. In order to understand the mechanism by which erbB-2 ECD inhibited colony formation of SK-Br-3 cells, growth-stimulatory doses of p75 were added to ECD-treated cells. The inhibitory effect of the ECD was reversed by the addition of stimulating doses of p75, suggesting that complex regulatory pathways may exist for p185^{erbB-2} (Figure 5).

### Summary

In brief, we have identified a novel polypeptide of 75 kDa that binds to the p185^{erbB-2} receptor. The effects of p75 on cells with very high levels of erbB-2 were similar to the reported effects of the other known ligand, gp30. In contrast to gp30, p75 appears to be specific for p185^{erb-2} receptor. Furthermore, we have provided evidence that cells that overexpress the erbB-2 receptor may also secrete one of its ligands, which is required for their proliferation, therefore implying an autocrine loop. We believe that manipulation of this and other erbB-2 ligands may turn out to have an important biological effect on growth of human neoplasia.

Modifications of the above-described modes for carrying out the invention that are obvious to persons of skill in medicine, immunology, hybridoma technology, pharmacology, and/or related fields are intended to be within the scope of the following claims.

All publications and patent applications mentioned in this specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claims.

## Claims

1. A substantially pure protein, wherein said protein:
reverses antiproliferative effect of soluble erbB-2 extracellular domain (ECD);
corresponds to a protein which is obtainable by elution of SK-BR-3 cancer cell conditioned media from an ECD affinity column at pH 3.0-3.5;
inhibits cell proliferation and colony formation of cells which overexpress erbB-2;
has apparent molecular weight as measured by SDS PAGE of about 75 kDa;
is capable of inducing phosphorylation of p185^{erbB-2}; and
does not bind EGFR.

2. A composition for use in inhibiting the growth of cells which overexpress the oncogene erbB-2 receptor comprising an amount of the substantially pure protein according to claim 1 which is effective to inhibit the growth of said cells.

3. A composition according to claim 2, wherein said cells are adenocarcinoma cells.

4. A composition for use in stimulating the growth of cells which overexpress the oncogene erbB-2 receptor comprising an amount of the substantially pure protein according to claim 1 which is sufficient to stimulate the growth of said cells.

5. A composition according to claim 4, wherein said cells are adenocarcinoma cells.

6. An antibody which specifically binds the protein of claim 1.

7. A composition for use in inhibiting the growth of cells which overexpress the oncogene erbB-2 receptor comprising treating said cells with an amount of antibody according to claim 6 which is effective to inhibit the growth of said cells.

8. A composition according to claim 7 wherein said antibody is conjugated to a therapeutic agent.

9. A composition for use in stimulating the growth of cells which overexpress oncogene erbB-2 receptor comprising treating said cells with an amount of antibody according to claim 6 which is sufficient to stimulate the growth of said cells.

10. The method of claim 9 wherein said antibody is conjugated to a therapeutic agent.

11. A method of detecting the presence of cells expressing the protein according to claim 1 in a patient comprising:
a. contacting a sample obtained from said patient with a detectably labelled antibody which specifically binds said protein for a time sufficient for binding between said antibody and said protein; and
b. detecting the presence of said protein bound to said antibody in said sample.

12. A method of preparing a substantially pure protein according to claim 1, comprising:
a. producing a cDNA library corresponding to mRNA from a cell line that expresses said protein and selecting clones form the library based on ability to hybridize with probes encoding a portion of the sequence of said protein;
b. transforming a host cell with DNA from the selection clones;
c. growing the transformed host cell in culture; and
d. recovering said protein from the culture.

13. A method for obtaining an isolated DNA molecule encoding the protein of claim 1, comprising:
a. constructing DNA probes which encode a portion of the sequence of said protein;
b. constructing a cDNA library from mRNA extracted from a cell line that expresses said protein;
c. selecting from said cDNA clones containing DNA which hybridizes to said DNA probes; and
d. isolating the DNA from the selected clones.

14. An isolated DNA molecule obtained by the method of claim 13.

## Patentansprüche

1. Ein im wesentlichen reines Protein, wobei das Protein:
die antiproliferative wirkung der löslichen extrazellulären erbB-2-Domäne (ECD) umkehrt;
einem Protein entspricht, das durch Elution von SK-BR-3-Krebszellen-Konditionierungsmedien von einer ECD-Affinitätssäule bei pH 3,0-3,5 erhältlich ist;
die Zellproliferation und Koloniebildung von Zellen inhibiert, welche erbB-2 überexprimieren;
ein scheinbares Molekulargewicht, gemessen durch SDS-PAGE, von ungefähr 75 kDa aufweist;
in der Lage ist, die Phosphorylierung von p185^{erbB-2} zu induzieren; und
EGFR nicht bindet.

2. Eine Zusammensetzung zur Verwendung beim Inhibieren des Wachstums von Zellen, die den Onkogen-erbB-2-Rezeptor überexprimieren, welche eine Menge des im wesentlichen reinen Proteins gemäß Anspruch 1 umfaßt, die wirksam ist, um das Wachstum der Zellen zu inhibieren.

3. Eine Zusammensetzung gemäß Anspruch 2, wobei die zellen Adenokarzinomzellen sind.

4. Eine zusammensetzung zur Verwendung beim Stimulieren des Wachstums von Zellen, die den Onkogen-erbB-2-Rezeptor überexprimieren, welche eine Menge des im wesentlichen reinen Proteins gemäß Anspruch 1 umfaßt, die ausreichend ist, um das Wachstum der Zellen zu stimulieren.

5. Eine Zusammensetzung gemäß Anspruch 4, wobei die Zellen Adenokarzinomzellen sind.

6. Ein Antikörper, welcher das Protein aus Anspruch 1 spezifisch bindet.

7. Eine Zusammensetzung zur Verwendung beim Inhibieren des Wachstums von Zellen, welche den Onkogen-erbB-2-Rezeptor überexprimieren, umfassend ein Behandeln der Zellen mit einer Menge des Antikörpers gemäß Anspruch 6, welche wirksam ist, um das Wachstum der Zellen zu inhibieren.

8. Eine Zusammensetzung gemäß Anspruch 7, wobei der Antikörper mit einem Heilmittel konjugiert ist.

9. Eine Zusammensetzung zur Verwendung beim Stimulieren des Wachstums von Zellen, welche den Onkogen-erbB-2-Rezeptor überexprimieren, umfassend ein Behandeln der Zellen mit einer Menge des Antikörpers gemäß Anspruch 6, welche ausreichend ist, um das wachstum der zellen zu stimulieren.

10. Das Verfahren aus Anspruch 9, wobei der Antikörper mit einem Heilmittel konjugiert ist.

11. Ein Verfahren zum Nachweisen des vorliegens von Zellen, welche das Protein gemäß Anspruch 1 exprimieren, in einem Patienten, umfassend:
a. In-Kontakt-Bringen einer Probe, die von dem Patienten erhalten wurde, mit einem nachweisbar markierten Antikörper, welcher das Protein spezifisch bindet, für ein Zeit, die ausreicht, damit eine Bindung zwischen dem Antikörper und dem Protein stattfinden kann; und
b. Nachweisen des Vorliegens des Proteins, das an den Antikörper gebunden ist, in der Probe.

12. Ein Verfahren zum Herstellen eines im wesentlichen reinen Proteins gemäß Anspruch 1, umfassend:
a. Herstellen einer cDNA-Bibliothek, welche *der* mRNA aus einer Zellinie entspricht, die das Protein exprimiert, und Selektionieren von Klonen aus der Bibliothek auf der Grundlage der Fähigkeit, mit Sonden zu hybridisieren, welche einen Teil der Sequenz des Proteins codieren;
b. Transformieren einer Wirtszelle mit DNA aus den Selektionsklonen;
c. Wachsenlassen der transformierten Wirtszelle in Kultur; und
d. Gewinnen des Proteins aus der Kultur.

13. Ein Verfahren zum Erhalten eines isolierten DNA-Moleküls, welches das Protein aus Anspruch 1 codiert, umfassend:
a. Konstruieren von DNA-Sonden, welche einen Teil der Sequenz des Proteins codieren;
b. Konstruieren einer cDNA-Bibliothek aus einer mRNA, welche aus einer Zellinie extrahiert wurde, die das Protein exprimiert;
c. Selektionieren der cDNA-Klone, welche DNA enthalten, die mit den DNA-Sonden hybridisiert; und
d. Isolieren der DNA aus den selektionierten Klonen.

14. Ein isoliertes DNA-Molekül, welches durch das Verfahren aus Anspruch 13 erhalten wurde.

## Revendications

1. Protéine essentiellement pure, où ladite protéine:
inverse l'effet antiprolifératif du domaine extra-cellulaire erbB-2 (ECD) soluble ;
correspond à une protéine pouvant être obtenue par élution, à partir d'une colonne d'affinité d'ECD à pH 3,0-3,5, de milieux conditionnés de cellules cancéreuses SK-BR-3 ;
inhibe la prolifération cellulaire et la formation de colonies de cellules qui surexpriment le domaine erB-2 ;
a une masse moléculaire apparente, mesurée par SDS-PAGE, d'environ 75 kDa ;
est capable d'induire une phosphorylation du p 185^{rbB2}; et
ne fixe pas l'EGFR.

2. Composition destinée à être utilisée pour inhiber la croissance de cellules qui surexpriment le récepteur de l'oncogène erbB-2, comprenant une quantité de la protéine essentiellement pure selon la revendication 1, qui a le pouvoir d'inhiber la croissance desdites cellules.

3. Composition selon la revendication 2, dans laquelle lesdites cellules sont des cellules d'adénocarcinome.

4. Composition destinée à être utilisée pour stimuler la croissance de cellules qui surexpriment le récepteur de l'oncogène erbB-2, comprenant une quantité de la protéine essentiellement pure selon la revendication 1, qui est suffisante pour stimuler la croissance desdites cellules.

5. Composition selon la revendication 4, dans laquelle lesdites cellules sont des cellules d'adénocarcinome.

6. Anticorps qui fixe d'une manière spécifique la protéine selon la revendication 1.

7. Composition destinée à être utilisée pour inhiber la croissance de cellules qui surexpriment le récepteur de l'oncogène erbB-2, qui consiste à traiter lesdites cellules avec une quantité de l'anticorps selon la revendication 6 qui a le pouvoir d'inhiber la croissance desdites cellules.

8. Composition selon la revendication 7, dans laquelle ledit anticorps est conjugué à un agent thérapeutique.

9. Composition destinée à être utilisée pour stimuler la croissance de cellules qui surexpriment le récepteur de l'oncogène erbB-2, qui consiste à traiter lesdites cellules avec une quantité de l'anticorps selon la revendication 6 qui est suffisante pour stimuler la croissance desdites cellules.

10. Procédé selon la revendication 9, dans lequel ledit anticorps est conjugué à un agent thérapeutique.

11. Procédé de détection de la présence de cellules exprimant la protéine selon la revendication 1, chez un patient, qui consiste :
a. à mettre en contact un échantillon obtenu à partir dudit patient avec un anticorps marqué d'une manière détectable qui fixe d'une manière spécifique ladite protéine pendant un laps de temps suffisant pour assurer une liaison entre ledit anticorps et ladite protéine ; et
b. à détecter la présence de ladite protéine fixée audit anticorps dans ledit échantillon.

12. Procédé de préparation d'une protéine essentiellement pure selon la revendication 1, qui consiste :
a. à produire une banque d'ADNc correspondant à l'ARNm provenant d'une lignée cellulaire qui exprime ladite protéine, et à sélectionner des clones dans la banque, sur la base de leur aptitude à s'hybrider à des sondes codant une portion de la séquence de ladite protéine ;
b. à transformer une cellule hôte à l'aide d'un ADN provenant des clones sélectionnés ;
c. à faire croître la cellule hôte transformée en culture ; et
d. à récupérer de la culture ladite protéine.

13. Procédé pour obtenir une molécule d'ADN isolée codant la protéine selon la revendication 1, qui consiste:
a. à synthétiser des sondes à ADN qui codent une portion de la séquence de ladite protéine ;
b. à synthétiser une banque d'ADNc à partir d'un ARNm extrait d'une lignée cellulaire qui exprime ladite protéine;
c. à sélectionner à partir dudit ADNc des clones contenant un ADN, qui s'hybride auxdites sondes à ADN ; et
d à isoler l'ADN des clones sélectionnés.

14. Molécule d'ADN isolée obtenue par le procédé selon la revendication 13.
